# EUROPÄISCHE PATENTSCHRIFT

(11) **EP 2 655 488 B1**
(45) Veröffentlichungstag und Bekanntmachung des Hinweises auf die Patenterteilung: **03.12.2014**
(21) Anmeldenummer: 11802914.9
(22) Anmeldetag: 19.12.2011
(51) Int. Cl.: C08J 5/02, C07C 225/06, C11D 3/37, C08L 83/04, C08J 3/03

(54) **ZUSAMMENSETZUNGEN ENTHALTEND QUATVERBINDUNGEN UND ORGANOPOLYSILOXANE**
COMPOSITIONS COMPRISING QUAT COMPOUNDS AND ORGANOPOLYSILOXANES
COMPOSITIONS CONTENANT DES COMPOSÉS QUATERNAIRES ET DES ORGANOSILOXANES

(30) Priorität: 21.12.2010 DE 102010063696
(43) Veröffentlichungstag der Anmeldung: 30.10.2013
(73) Patentinhaber: Wacker Chemie AG, 81737 München (DE)
(72) Erfinder: HERZIG, Christian, 83329 Waging (DE); BECKER, Richard, 84489 Burghausen (DE); BAUMANN, Eva, 83661 Lenggries (DE)
(74) Vertreter: Deffner-Lehner, Maria
(86) Internationale Anmeldenummer: PCT/EP2011/073246
(87) Internationale Veröffentlichungsnummer: WO 2012/084830

(56) Entgegenhaltungen:
- WO-A1-2012/016837
- DE-A1- 19 505 751

## Beschreibung

Die Erfindung betrifft Zusammensetzungen enthaltend Quatverbindungen und Organopolysiloxane und deren Verwendung in Weichspülern.

EP 1 259 672 B1 beschreibt die Verwendung von Alkyl-Keten-Dimer-Dispersionen zur Antiknitterausrüstung von Textilien. Beschrieben sind auch kationische Emulgatoren, die aus Alkyl-Keten-Dimer und Polyethylenimin in einer Teilumsetzung hergestellt werden. Bis auf diese Teilumsetzungsprodukte, in denen einige Amingruppen des Polyethylenimin amidiert werden, bleibt das dispergierte Alkyl-Keten-Dimer intakt und wird als solches auf die Faser gebracht. Eine Quaternierung des teilumgesetzten Polyethylenimin-Emulgators ist nicht beschrieben und wäre auch wegen des hohen Prozentsatzes von prim. und sek. Aminogruppen kaum wirtschaftlich durchzuführen.

Alkyl-Keten-Dimer-Dispersionen als Textilhilfsmittel sind in folgenden Dokumenten beschrieben:
CH 388246 beschreibt die Verwendung von Formulierungen, die den Ketenweichmacher "Aquapel" 380 (Hercules Powder Co.) zur Imprägnierung und Hydrophobierung von Textilien enthalten. Auch in US 5,028,236 werden Alkyl-Keten-Dimere zur Hydrophobierung von Wolle und synthetischen Polyamidfasern eingesetzt. Mit demselben Ziel werden in WO 96/26318 Alkyl-Keten-Dimer-Dispersionen als Leimungsmittel für Papier, also zur kontrollierten Hydrophobierung, verwendet.

WO 2009/121751 beschreibt die Umsetzung von Aminosiloxanen mit Alkyl-Keten-Dimeren zur Herstellung von Wachsen mit sehr hohem Polydimethylsiloxan-Gehalt. Quaternäre Verbindungen können damit nicht hergestellt werden.

US 2009/247629 A1 beschreibt die Herstellung polymerer organischer Quatverbindungen, die sich durch Veresterung von Dimersäure mit Dimethylaminoalkanol und nachfolgendem Umsatz mit Polyetherepichlorhydrinen herstellen lassen. Die Synthesefolge ist relativ aufwändig und bei hoher Temperatur (200°C) über viele Stunden (3-8 h) auszuführen, also wenig attraktiv.

Kuo-Yann Lai (Editor) beschreibt in "Liquid Detergents", Second Edition, "S. 509, CRC-Press 2006, dass die zusätzliche Einformulierung von "PDMS" in Weichmacherformulierungen auf Basis von Esterquats die Benetzung von damit behandeltem Bauwollgewebe überraschend verbessert.

Ähnliches ist in EP 869 168 A1 offenbart, worin Formulierungen beschrieben sind, die zusätzlich noch hydrophile Emulgatoren enthalten und deren Esterquats mit weiteren Ethylenoxygruppen modifiziert sein können.

Es bestand die Aufgabe Quatverbindungen, d. h. Verbindungen, die mindestens eine quartäre Ammoniumgruppe enthalten, bereitzustellen, die in einem einfachen Verfahren schnell und nahezu quantitativ hergestellt werden können und die einen längerkettigen Kohlenwasserstoffrest, vorzugsweise Fettsäurerest, aufweisen. Weiterhin bestand die Aufgabe Zusammensetzungen enthaltend Quatverbindungen und Organopolysiloxane bereitzustellen, die in Weichspülern eingesetzt werden können, wobei die damit behandelten Textilien einen verbesserten Weichgriff und eine verbesserte Hydrophilie aufweisen. Die Aufgabe wird durch die Erfindung gelöst.

Gegenstand der Erfindung sind Zusammensetzungen enthaltend
(1) β-Ketocarbonylquatverbindungen, die eine oder mehrere β-Ketocarbonylgruppen der allgemeinen Formel

   R-CH₂-(C=O)-CHR-(C=O)- (I)

   und eine oder mehrere quartäre Ammoniumgruppen enthalten, wobei R gleich oder verschieden sein kann und einen aliphatischen Kohlenwasserstoffrest mit 6 bis 28 C-Atomen bedeutet, und
(2) bei 25°C und 1020 hPa flüssige Organopolysiloxane oder Siloxancopolymere.

Der Rest R ist vorzugsweise ein aliphatischer Kohlenwasserstoffrest 10 bis 26 C-Atomen, bevorzugt 12 bis 20 C-Atomen.

Quartäre Ammoniumgruppen (quaternary ammonium groups) sind Derivate der Ammoniumgruppe, bei der alle vier Wasserstoffatome durch vier N-C-gebundene (ggf. substituierte) Kohlenwasserstoffgruppen, wie Alkylgruppen, ersetzt sind.

Vorzugsweise ist die β-Ketocarbonylgruppe der Formel (I) an einen Rest Y gebunden, wobei
- Y: einen zweiwertigen Rest der Formel -O-, -NH-, -NR¹-, bevorzugt -NH-, -NR¹-, oder einen dreiwertigen Rest der Formel =N- bedeutet, und
- R¹: einen einwertigen Kohlenwasserstoffrest mit 1 bis 30 C-Atomen, vorzugsweise 1 bis 18 C-Atomen, bedeutet.

Die erfindungsgemäßen β-Ketocarbonylquatverbindungen enthalten vorzugsweise ein oder zwei quartäre N-Atome, bevorzugt ein quartäres N-Atom.
Sie haben ein Molekulargewicht (Mₙ) von vorzugsweise mindestens 300 Dalton, bevorzugt 500 bis 2000 Dalton. Die bevorzugte Ladungsdichte liegt bei 0,5 bis 2,0 mEquiv. N⁺/g.

Bevorzugt als β-Ketocarbonylquatverbindungen sind solche der allgemeinen Formel

[R³R⁴R⁵N⁽⁺⁾-R²-]ₐY-Z X⁽⁻⁾ (II),

wobei
- a: 1 oder 2 ist, mit der Maßgabe, dass wenn a=1, Y ein zweiwertiger Rest ist und wenn a=2, Y ein dreiwertiger Rest ist,
- Y: einen zweiwertigen Rest der Formel -O-, -NH- , -NR¹-, oder einen dreiwertigen Rest der Formel =N- bedeutet,
- X⁽⁻⁾: Gegenion zu der positiven Ladung am quartären Stickstoffatoms ist,
- Z: eine β-Ketocarbonylgruppe der Formel R-CH₂-(C=O)-CHR-(C=O)- (I) bedeutet,
- R: die oben dafür angegebene Bedeutung hat,
- R¹: einen einwertigen Kohlenwasserstoffrest mit 1 bis 30 C-Atomen, vorzugsweise 1 bis 18 C-Atomen, bedeutet,
- R²: einen zweiwertigen C₁-C₁₈-Kohlenwasserstoffrest, der ein oder mehrere separate Sauerstoffatome enthalten kann, bedeutet,
- R³: gleich oder verschieden ist und einen einwertigen Kohlenwasserstoffrest mit 1 bis 30 C-Atomen, vorzugsweise 1 bis 18 C-Atomen, bedeutet,
- R⁴: gleich R³ ist oder einen Rest der Formel -R¹-Y-Z oder -R¹-N⁽⁺⁾R³₂R⁵ X⁽⁻⁾ bedeutet,
oder R³ und R⁹ zusammen oder zwei Reste R³ zusammen einen zweiwertigen C₃-C₁₂-Kohlenwasserstoffrest, der gegebenenfalls ein O-Atom oder N-Atom enthalten kann, bedeuten und
- R⁵: einen einwertigen Kohlenwasserstoffrest mit 1 bis 18 C-Atomen, vorzugsweise 1 bis 12 C-Atomen, bedeutet.

Die erfindungsgemäßen β-Ketocarbonylquatverbindungen werden hergestellt,
indem in einem ersten Schritt
Alkyl-Keten-Dimere (a) der allgemeinen Formel 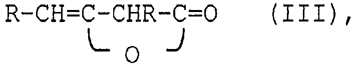 wobei R die oben dafür angegebene Bedeutung hat, mit
Aminoverbindungen (b), die mindestens eine tertiäre Aminogruppe und mindestens eine protische Gruppe ausgewählt aus der Gruppe der Formeln -OH, -NH₂, -NHR¹ und -NH-, vorzugsweise -NH₂, -NHR¹ und -NH-, enthalten, wobei R¹ die oben dafür angegebene Bedeutung hat,
umgesetzt werden, wobei tertiäre Aminogruppen aufweisende Verbindungen (c) erhalten werden,
und in einem zweiten Schritt,
die tertiären Stickstoffatome aus den im ersten Schritt erhaltenen Verbindungen (c) mit Alkylierungsmitteln (d) teilweise oder vollständig quaterniert werden.

Vorzugsweise ist R ein Alkylrest, bevorzugt ein linearer Alkylrest, mit mindestens 6 bis 28 C-Atomen, bevorzugt 10 bis 26 C-Atomen und besonders bevorzugt 12 bis 20 C-Atomen.

Bevorzugt ist R¹ ist ein Alkylrest mit 1 bis 18 C-Atomen.

Vorzugsweise sind R³ und R⁴ Alkylreste oder Cycloalkylreste. Bevorzugt ist mindestens ein Rest aus der Gruppe R³, R⁴, R⁵ ein Methyl- oder Ethylrest.

Beispiele für Reste R sind Alkylreste, wie Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste; Alkenylreste, wie der Vinyl-, 5-Hexenyl-, Cyclohexenyl-, 1-Propenyl-, Allyl-, 3-Butenyl- und 4-Pentenylrest; und Alkinylreste, wie der Ethinyl-, Propargyl- und 1-Propinylrest.

Beispiele für Kohlenwasserstoffreste R¹ sind Alkylreste, wie der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; Cycloalkylreste, wie Cyclopentyl-, Cyclohexyl-, Cycloheptyl- und Methylcyclohexylreste; Alkenylreste, wie der Vinyl-, 5-Hexenyl-, Cyclohexenyl-, 1-Propenyl-, Allyl-, 3-Butenyl- und 4-Pentenylrest; Alkinylreste, wie der Ethinyl-, Propargyl- und 1-Propinylrest; Arylreste, wie der Phenyl-, Naphthyl-, Anthryl- und Phenanthrylrest; Alkarylreste, wie o-, m-, p-Tolylreste, Xylylreste und Ethylphenylreste; und Aralkylreste, wie der Benzylrest, der α- und der β-Phenylethylrest.

Beispiele für Kohlenwasserstoffreste R¹ gelten im vollen Umfang für Kohlenwasserstoffreste R³ und R⁴.

Beispiele für zweiwertige Kohlenwasserstoffreste R² sind Alkylenreste der Formel -CH₂-, -CH₂CH₂-, -CH₂CH₂CH₂-, -CH₂CH₂CH₂CH₂-, -CH₂CH(CH₃)-, und Beispiele für zweiwertige Kohlenwasserstoffreste R² , die ein oder mehrere separate Sauerstoffatome enthalten können, sind solche der Formel -CH₂CH₂OCH₂CH₂-, -CH₂CH(CH₃)OCH₂CH(CH₃) -, CH₂CH(CH₃)OCH₂CH₂-, -CH₂CH₂OCH₂CH₂OCH₂CH₂-.

Beispiele für Alkyl-Keten-Dimere (a) sind solche, die sich von C₈- bis C₃₀-Carbonsäuren ableiten. Die Kohlenwasserstoffreste R können linear, verzweigt oder cyclisch sein, lineare Reste sind bevorzugt. Sie können gesättigt, sowie einfach oder mehrfach ungesättigt sein, wobei gesättigt lineare Kohlenwasserstoffreste bevorzugt sind. Bevorzugte Alkyl-Keten-Dimere (a) leiten sich von C₁₂- bis C₂₈-Carbonsäuren ab, wobei solche auf Basis von C₁₄- bis C₂₂-Carbonsäuren besonders bevorzugt sind. Die Herstellung von Alkyl-Keten-Dimere (a) aus Carbonsäuren ist in US 5,028,236 beschrieben. Allgemein gilt, dass die Anzahl der C-Atome im Rest R der um 2 verminderten Anzahl der C-Atome der den Alkyl-Keten-Dimeren (a) zugrunde liegenden Carbonsäuren entspricht. Anstatt eines Alkyl-Keten-Dimeren mit definierter Kettenlänge R können auch Gemische aus verschiedenen Alkyl-Keten-Dimere (a) mit jeweils verschieden langen R eingesetzt werden, wie sie z. B. aus Carbonsäuregemischen erhalten werden, die aus natürlichen Quellen stammen. Geeignete Fettalkyldiketene sind in EP 1 259 672 B1, Seite 2, Zeilen 52-57 beschrieben.

Die im ersten Schritt des Verfahrens eingesetzten Aminoverbindungen (b) enthalten mindestens eine tertiäre Aminogruppe und zusätzlich noch mindestens eine protische Gruppe ausgewählt aus der Gruppe der Formeln -OH, -NH₂, -NHR und -NH-. Diese protische Gruppe dient dazu, in einer ersten Stufe mit dem Alkyl-Keten-Dimer (a) zu reagieren und damit mindestens eine tertiäre Aminogruppe an das Alkyl-Keten-Dimer zu binden. Die im ersten Schritt des erfindungsgemäßen Verfahrens erhaltene Verbindung (c) enthält also mindestens eine tertiäre Aminogruppe. Im zweiten Schritt des erfindungsgemäßen Verfahrens werden die so gebundenen tertiären Aminogruppen mit Alkylierungsmitteln (d) teilweise oder vollständig quaterniert.

Bevorzugt werden im ersten Verfahrensschritt als Aminoverbindungen (b) solche der allgemeinen Formel

[R³R^{4'}N-R²-]ₐY-H (IV)

eingesetzt, wobei
- R⁴': gleich R³ ist oder einen Rest der Formel -R¹-Y-H oder -R¹-NR³₂ bedeutet, und
- a, R¹, R², R³ und Y: die oben dafür angegebene Bedeutung haben.

Beispiele für tertiäre Aminoverbindungen (b), die noch mindestens eine weitere protische Stickstofffunktion tragen sind:
3-Dimethylaminopropylamin,
3-Dimethylaminopropyl-butylamin,
Bis-(3-dimethylaminopropyl)amin,
Bis-(3-aminopropyl)-methylamin,
3-Diethylaminopropyl-1-methylamin,
2-Diethylaminoethylamin,
3-Aminopropyl-3-dimethylaminopropylamin.

Beispiele für tertiäre Aminoverbindungen (b), die noch mindestens eine weitere OH-Gruppe tragen sind:
2-Diethylaminoethanol,
2-Dimethylaminoethanol,
3-Dimethylaminopropanol,
2-Dimethylamino-1-methylethanol,
Bis-(hydroxyethyl)-methylamin,
2-Cyclohexylaminoethanol,
2-Morpholinoethanol,
2-((2-Dimethylaminoethoxy)ethanol,
N,N,N-Trimethyl-N-hydroxyethyl-bisaminoethylether,
Bis-(3-dimethylaminopropyl)-hydroxyethylamin,
3-Dimethylaminopropyl-bis-(2-hydroxyethyl)amin
3-Dimethylaminopropyl-2-hydroxyethylamin,
Tris-(2-hydroxyethyl)amin.

Weitere Beispiele für tertiäre Aminoverbindungen (b) sind die genannten Aminoverbindungen in ihrer ethoxylierten oder propoxylierten Form.

Geeignet als im zweiten Verfahrensschritt eingesetzte Alkylierungsmittel (d) sind alle Verbindungen, die quaternierend auf tertiäre Aminogruppen wirken.

Vorzugsweise werden als Alkylierungsmittel (d) solche der Formel

R⁵-X (V),

eingesetzt, wobei
- R⁵: die oben dafür angegebene Bedeutung hat, und
- X: ein Rest, der bei der Alkylierung der tertiären Stickstoffatome in den Verbindungen (c) ein Gegenion X⁻ zu der positiven Ladung am quartären Stickstoffatom bildet.

Bevorzugt ist R⁵ ein linearer, verzweigter oder cyclischer Alkylrest mit 1 bis 6 Kohlenstoffatomen.
Beispiele für Reste R⁵ sind der Methyl-, Ethyl-, Propyl-, Isopropyl-, Butyl-, Isobutyl und der Cyclohexylrest.

Beispiele für geeignete Alkylierungsmittel (d) sind Dialkylsulfate, wie Dimethylsulfat und Diethylsulfat, Sulfonsäureester, wie p-Toluolsulfonsäuremethylester , p-Toluolsulfonsäureethylester, p-Toluolsulfonsäurepropylester, sowie auch Benzylverbindungen, wie Benzylchlorid , Benzylbromid und Benzyljodid. Besonders bevorzugt sind Dimethylsulfat, Diethylsulfat und p-Toluolsulfonsäuremethylester.

Beispiele für Gegenionen X⁻ zur positiven Ladung am quartären Stickstoffatom sind
CH₃SO₄⁻
CH₃CH₂SO₄⁻
C₆H₅SO₃⁻
p-CH₃(C₆H₄)SO₃⁻
CH₃SO₃⁻
C₄H₉SO₃⁻
C₈H₁₇SO₃⁻
Cl⁻, Br⁻ und J⁻.

Das zweistufige Verfahren kann in zwei getrennten Syntheseschritten erfolgen, wie auch bevorzugt in einem Tandemverfahren in einer Reaktionsanlage. Da die Alkyl-Keten-Dimere (a) bei 25°C meist fest sind, erfolgt der erste Verfahrensschritt vorzugsweise bei erhöhter Temperatur im Bereich von 40 bis 140°C, bevorzugt bei 50 bis 120°C.
Bevorzugt findet der zweite Verfahrensschritt bei 60 bis 140°C statt. Falls erwünscht, lässt sich die gesamte Synthese ohne zwischenzeitliches Abkühlen vorteilhaft in einem Zug durchfahren.

Das Verfahren zur Herstellung der erfindungsgemäßen β-Keto-carbonylquatverbindungen (1) wird vorzugsweise beim Druck der umgebenden Atmosphäre, also etwa bei 1020 hPa durchgeführt, kann aber auch bei höheren oder niedrigeren Drücken durchgeführt werden.

In ersten Schritt des Verfahrens wird Alkyl-Keten-Dimer (a) in Mengen von vorzugsweise 0,8 bis 1,2 Mol Diketen, bevorzugt 0,9 bis 1,1 Mol Diketen, je Mol protische Gruppe in der Aminoverbindung (b) eingesetzt.
Alkyl-Keten-Dimere (a) werden besonders bevorzugt equimolar zu den in den Aminoverbindungen (b) enthaltenen protischen Gruppen eingesetzt. Zu berücksichtigen ist dabei, dass (a) selten reines Alkyl-Keten-Dimer ist, und meist mit einer Reinheit von 85 bis 95% im Handel erhältlich ist.

Im zweiten Schritt des Verfahrens wird Alkylierungsmittel (d) vorzugsweise in Mengen von 0,8 bis 1,0 Mol, bevorzugt 0,9 bis 1,0 Mol, je Mol tertiäres Stickstoffatom in der Verbindung (c) eingesetzt. Zu berücksichtigen ist auch hier die Reinheit des Alkylierungsmittels, so wie auch die der übrigen Komponenten. Sollten diese Verunreinigungen enthalten, die mit dem Alkylierungsmittel ebenfalls reagieren, können zur vollständigen Quaternierung der tert. Stickstoffatome auch mehr als die bevorzugten 1,0 Mol Alkylierungsmittel je Mol tert. Stickstoffatom erforderlich sein.

Das Verfahren zur Herstellung der erfindungsgemäßen β-Keto-carbonylquatverbindungen (1) hat gegenüber dem Verfahren zur Herstellung der bekannten Esterquats, wie sie z. B. in US 2009/0247629 A1 beschrieben sind, den Vorteil, dass die Amidierung im ersten Verfahrensschritt in weniger als 30 Minuten bei 60°C verläuft gegenüber US 2009/0247629 A1, in der die Veresterung, die noch dazu immer unvollständig ist, bei 200°C über viele Stunden (3-8 Stunden) erfolgt und die anschließende Umsetzung des Esteramins zur Esterquatverbindung weitere 6-9 Stunden bei 90°C benötigt.

Ein weiterer Vorteil ist, dass erfindungsgemäß eine nahezu quantitative Quaternierung erzielt wird gegenüber einem 60-90% Quaternierungsgrad bei der Herstellung der Esterquats gemäß Stand der Technik.
Dies bedeutet, dass keine Überschüsse an Alkylierungsmittel (d) eingesetzt werden müssen, um eine nahezu quantitative Quaternierung zu erreichen, und daher im Endprodukt keine toxischen Alkylierungsmittel (d) enthalten sind. Auch bei einer absichtlich knappen Unterdosierung des Alkylierungsmittels (d) werden immer noch sehr hohe Ausbeuten erzielt.
Damit ist eine sehr hohe Aktivkonzentration realisierbar und damit entsprechend wenig Belastung der Abwässer durch Nebenprodukte.

Ein weiterer Vorteil ist, das die erfindungsgemäßen β-Ketocarbonylquatverbindungen biologisch abbaubar sind.

Als Organopolysiloxane (2) in den erfindungsgemäßen Zusammensetzungen können alle bei 25°C und 1020 hPa flüssige Organopolysiloxane eingesetzt werden, die einen weichmachenden Effekt bewirken können. Dies können reine Organopolysiloxane sein, die ausschließlich aus Siloxaneinheiten aufgebaut sind, oder auch Copolymere mit organischen Polymeren, also Copolymere die eine Blockstruktur von Siloxan- und organischen Polymereinheiten aufweisen. Bevorzugt sind aber Organopolysiloxane, besonders solche mit die weitgehend linear sind.

Diese linearen Organopolysiloxane setzten sich im Wesentlichen aus Kettenbausteinen, wie Diorganosiloxaneinheiten, vorzugsweise Dimethylsiloxaneinheiten, und endständige Siloxaneinheiten zusammen. Der Anteil an Diorganosiloxaneinheiten, bevorzugt Dimethylsiloxaneinheiten, beträgt vorzugsweise mindestens 90 Gew.-%, bevorzugt mindestens 95 Gew.-%, besonders bevorzugt mindestens 98 Gew.-%, jeweils bezogen auf das mittlere Gesamtgewicht der Organopolysiloxane (2). Bei linearen Organopolysiloxanen, die mit Triorganosiloxaneinheiten, bevorzugt Trimethylsiloxaneinheiten, terminiert sind, beträgt das Verhältnis von Diorganosiloxan- zu Triorganosiloxaneinheiten, bevorzugt Dimethylsiloxan- zu Trimethylsiloxaneinheiten vorzugsweise mindestens 20 : 1, bevorzugt 50 : 1, besonders bevorzugt 100 : 1.

Beispiele für Organopolysiloxane (2) sind Dialkylpolysiloxane, wie Dimethylpolysiloxane, die Alkyl-, Alkoxy- oder Hydroxyendgruppen aufweisen, Dialkylpolysiloxane, die primäre, sekundäre tertiäre und/ oder quartäre Aminogruppen aufweisen, Copolymere aus Siloxaneinheiten und organischen Polymereinheiten, wie Polyether, Polyester, Polycarbonate und Polyurethane/Polyharnstoffe.
Enthalten die Organpolysiloxane (2) auch Aminogruppen, so ist deren Konzentration bevorzugt höchstens 0,3 mEquiv. N/g.

Bevorzugt werden als Organopolysiloxane (2) solche der allgemeinen Formel

R^{''}R'₂SiO(R'₂SiO)ₘSiR'₂R^{''} (VI)

eingesetzt, wobei
R' einen Kohlenwasserstoffrest mit 1 bis 18 C-Atomen, vorzugsweise einen C₁-C₁₈-Alkylrest, bevorzugt einen C₁-C₄-Alkylrest, bedeutet,
R" einen Rest R', einen Hydroxyrest oder einen C₁-C₁₈-Alkoxyrest bedeutet und
m eine ganze Zahl von 20 bis 5000, vorzugsweise 100 bis 2000, bevorzugt 300 bis 2000, ist.

Beispiele für Alkylreste R' sind der Methyl-, Ethyl-, n-Propyl-, iso-Propyl-, 1-n-Butyl-, 2-n-Butyl-, iso-Butyl-, tert.-Butyl-, n-Pentyl-, iso-Pentyl-, neo-Pentyl-, tert.-Pentylrest, Hexylreste, wie der n-Hexylrest, Heptylreste, wie der n-Heptylrest, Octylreste, wie der n-Octylrest und iso-Octylreste, wie der 2,2,4-Trimethylpentylrest, Nonylreste, wie der n-Nonylrest, Decylreste, wie der n-Decylrest, Dodecylreste, wie der n-Dodecylrest, und Octadecylreste, wie der n-Octadecylrest; wobei der Methylrest besonders bevorzugt ist.

Wenn R'' ein Alkoxyrest ist, ist es vorzugsweise ein C₁-C₄-Alkoxyrest.

Die erfindungsgemäß eingesetzten Organopolysiloxane (2) haben vorzugsweise eine Viskosität von 50 bis 100 000mPa^{·}s (25°C), bevorzugt 100 bis 20 000 mPa˙s (25°C).

Vorzugsweise sind die erfindungsgemäßen Zusammensetzungen wässrige Dispersionen, wie wässrige Emulsionen.

Bevorzugt als erfindungsgemäße Zusammensetzungen sind solche, die
(A) Dispersionen von β-Ketocarbonylquatverbindungen enthaltend
   (1) β-Ketocarbonylquatverbindungen,
   (3) ggf. organische Lösungsmittel,
   (4) ggf. Emulgator und
   (5) Wasser
   und
(B) Dispersionen von Organopolysiloxanen oder Siloxancopolymeren enthaltend
   (2) bei 25°C und 1020 hPa flüssige Organopolysiloxane oder Siloxancopolymere,
   (4') ggf. Emulgator und
   (5') Wasser,
enthalten, mit der Maßgabe, dass die Zusammensetzungen entweder als 2-Komponenten-Zusammensetzungen oder als Mischungen von (A) mit (B) in Form von 1-Komponenten-Zusammensetzung vorliegen.

Da die erfindungsgemäßen Quatverbindungen (1) bei 25°C meist wachsartige Feststoff sind, können bei Herstellung der Dispersionen (A) die Quatverbindungen (1) zur besseren Verarbeitbarkeit mit organischen Lösungsmitteln (3) verdünnt werden, analog wie bei den Esterquats des Standes der Technik. In diese Verdünnungen wird, vorzugsweise unter leichtem Erwärmen bei 30 bis 80°C, Wasser ggf. in Gegenwart von Emulgatoren, falls die erfindungsgemäße Quatverbindung (1) nicht selbstdispergierend ist, eingerührt.

Um die Dispersionen über längere Zeit haltbar zu machen, können sie Konservierungsmittel enthalten.

Beispiele für organische Lösungsmittel (3), die zur Herstellung der Dispersionen (A), verwendet werden können, sind Alkohole, wie Methanol, Ethanol, n-Propanol und iso-Propanol, und Glycolether, wie Diethylenglycolmonobuthylether (Butyldiglycol).

Bei den Dispersionen (A) werden organische Lösungsmittel (3) in Mengen von vorzugsweise 0 bis 50 Gewichtsteile, bevorzugt 10 bis 50 Gewichtsteile, jeweils bezogen auf 100 Gewichtsteile β-Ketocarbonylquatverbindungen (1), eingesetzt.

Zur Herstellung der Dispersionen (A) können Emulgatoren (4) mitverwendet werden. Als Emulgatoren können nicht-ionische, kationische und anionische Emulgatoren, bevorzugt nichtionische Emulgatoren, sowohl einzeln als auch als Mischungen verschiedener Emulgatoren eingesetzt werden.

Als anionische Emulgatoren können eingesetzt werden:
1. Alkylsulfate, besonders solche mit einer Kettenlänge von 8 bis 18 C-Atomen, Alkyl- und Alkarylethersulfate mit 8 bis 18 C-Atomen im hydrophoben Rest und 1 bis 40 Ethylenoxid (EO)- bzw. Propylenoxid(PO)einheiten.
2. Sulfonate, besonders Alkylsulfonate mit 8 bis 18 C-Atomen, Alkylarylsulfonate mit 8 bis 18 C-Atomen, Tauride, Ester und Halbester der Sulfobernsteinsäure mit einwertigen Alkoholen oder Alkylphenolen mit 4 bis 15 C-Atomen; gegebenenfalls können diese Alkohole oder Alkylphenole auch mit 1 bis 40 EO-Einheiten ethoxyliert sein.
3. Alkali- und Ammoniumsalze von Carbonsäuren mit 8 bis 20 C-Atomen im Alkyl-, Aryl-, Alkaryl- oder Aralkylrest.
4. Phosphorsäureteilester und deren Alkali- und Ammoniumsalze, besonders Alkyl- und Alkarylphosphate mit 8 bis 20 C-Atomen im organischen Rest, Alkylether- bzw. Alkaryletherphosphate mit 8 bis 20 C-Atomen im Alkyl- bzw. Alkarylrest und 1 bis 40 EO-Einheiten.

Als nichtionische Emulgatoren eignen sich besonders:
5. Polyvinylalkohol, der noch 5 bis 50 %, vorzugsweise 8 bis 20 % Vinylacetateinheiten aufweist, mit einem Polymerisationsgrad von 500 bis 3000.
6. Alkylpolyglycolether, vorzugsweise solche mit 8 bis 40 EO-Einheiten und Alkylresten von 8 bis 20 C-Atomen.
7. Alkylarylpolyglycolether, vorzugsweise solche mit 8 bis 40 EO-Einheiten und 8 bis 20 C-Atomen in den Alkyl- und Arylresten.
8. Ethylenoxid/Propylenoxid(EO/PO)-Blockcopolymere, vorzugsweise solche mit 8 bis 40 EO- bzw. PO-Einheiten.
9. Additionsprodukte von Alkylaminen mit Alkylresten von 8 bis 22 C-Atomen mit Ethylenoxid oder Propylenoxid.
10. Fettsäuren mit 6 bis 24 C-Atomen.
11. Alkylpolyglykoside der allgemeinen Formel R*-O-Z_{O}, worin R* einen linearen oder verzweigten, gesättigten oder ungesättigten Alkylrest mit im Mittel 8 - 24 C-Atomen und Z_{O} einen Oligoglykosidrest mit im Mittel o = 1 - 10 Hexose- oder Pentoseeinheiten oder Gemischen davon bedeuten.
12. Naturstoffe und deren Derivate, wie Lecithin, Lanolin, Saponine, Cellulose; Cellulosealkylether und Carboxyalkylcellulosen, deren Alkylgruppen jeweils bis zu 4 Kohlenstoffatome besitzen.
13. Polare Gruppen enthaltende lineare Organo(poly)siloxane, insbesondere solche mit Alkoxygruppen mit bis zu 24 C-Atomen und/oder bis zu 40 EO- und/oder PO-Gruppen.

Als kationische Emulgatoren können eingesetzt werden:
14. Salze von primären, sekundären und tertiären Fettaminen mit 8 bis 24 C-Atomen mit Essigsäure, Schwefelsäure, Salzsäure und Phosphorsäuren.
15. Quarternäre Alkyl- und Alkylbenzolammoniumsalze, insbesondere solche, deren Alkylgruppen 6 bis 24 C-Atome besitzen, insbesondere die Halogenide, Sulfate, Phosphate und Acetate.
16. Alkylpyridinium-, Alkylimidazolinium- und Alkyloxazoliniumsalze, insbesondere solche, deren Alkylkette bis zu 18 C-Atome besitzt, speziell die Halogenide, Sulfate, Phosphate und Acetate.

Als Ampholytische Emulgatoren können eingesetzt werden:
17. Langkettig substituierte Aminosäuren, wie N-Alkyl-di-(aminoethyl-)glycin oder N-Alkyl-2-aminopropionsäuresalze.
18. Betaine, wie N-(3-Acylamidopropyl)-N,N-dimethylammonium-salze mit einem C₈-C₁₈-Acylrest und Alkyl-imidazolium-Betaine.

Bevorzugt als Emulgatoren sind nichtionische Emulgatoren, insbesondere die vorstehend unter 6. aufgeführten Alkylpolyglycolether, die unter 9. aufgeführten Additionsprodukte von Alkylaminen mit Ethylenoxid oder Propylenoxid, die unter 11. aufgeführten Alkylpolyglykoside und der unter 5. aufgeführte Polyvinylalkohol.

Bei der Herstellung der Disperionen (A) werden Emulgatoren (4) in Mengen von vorzugsweise 0 bis 25 Gewichtsteilen, bevorzugt 1 bis 18 Gewichtsteilen, jeweils bezogen auf 100 Gewichtsteile β-Ketocarbonylquatverbindungen (1), eingesetzt.

Bei der Herstellung der Disperionen (A) wird Wasser (5), in Mengen von vorzugsweise 50 bis 600 Gewichtsteilen, besonders bevorzugt 100 bis 500 Gewichtsteilen, jeweils bezogen auf 100 Gewichtsteile β-Ketocarbonylquatverbindungen (1), eingesetzt.

Die Herstellung der erfindungsgemäßen Dispersion (B) erfolgt durch Mischen, bevorzugt intensives Mischen von den Organopolysiloxanen (2) mit Wasser (5') gegebenenfalls von Emulgatoren (4').
Technologien zur Herstellung von Emulsionen von Organopolysiloxanen sind bekannt. So kann das intensive Mischen in Rotor-Stator-Rührvorrichtungen, Kolloidmühlen oder in Hochdruckhomogenisatoren erfolgen.

Als Emulgatoren (4') können die Emulgatoren (4), die zur Herstellung der Dispersionen (A) eingesetzt werden, verwendet werden. Bevorzugt sind nicht-ionische Emulgatoren.

Bei der Herstellung der Disperionen (B) werden Emulgatoren (4') in Mengen von vorzugsweise 0 bis 25 Gewichtsteilen, bevorzugt 1 bis 18 Gewichtsteilen, jeweils bezogen auf 100 Gewichtsteile Organopolysiloxane (2), eingesetzt.

Bei der Herstellung der Dispersionen (B) wird Wasser (5'), in Mengen von vorzugsweise 10 bis 150 Gewichtsteilen, besonders bevorzugt 20 bis 100 Gewichtsteilen, jeweils bezogen auf 100 Gewichtsteile Organopolysiloxane (2), eingesetzt.

Die Dispersionen (A) und (B) werden in solchen Gewichtsmengen eingesetzt, dass das Mengenverhältnis der β-Ketocarbonylquat-verbindung (1) zu dem Organopolysiloxan (2) vorzugsweise 1 : 99 bis 99 : 1 , bevorzugt 1 : 10 bis 10 : 1 , beträgt.

Gegenstand der Erfindung sind weiterhin Weichspülmittel enthaltend die erfindungsgemäßen Zusammensetzungen.

Die Disperionen (A) werden in den Weichspülmitteln in solchen Mengen eingesetzt, dass die β-Ketocarbonylquatverbindungen (1) in Mengen von 0,01 bis 20 Gew.-%, bezogen auf das Gesamtgewicht des Weichspülers, enthalten sind.

Die Disperionen (B) werden in den Weichspülmitteln in solchen Mengen eingesetzt, dass die Organopolysiloxane (2) in Mengen von 0,01 bis 20 Gew.-%, bezogen auf das Gesamtgewicht des Weichspülmittels, enthalten sind.

Die Dispersionen (A) und (B) können getrennt als 2-Komponentensysteme oder als Mischungen der Dispersionen (A) mit den Dispersionen (B) in Form von 1-Komponentensystemen vorliegen.

Die erfindungsgemäßen Weichspüler enthalten neben den β-Ketocarbonylquatverbindungen (1) und Organopolysiloxanen (2) bzw. neben den Dispersionen (A) und (B) übliche Bestandteile von Weichspülmittel.

Beispiele für weitere Bestandteile in den Weichspülern sind primäre Alkohole, wie Ethanol oder Isopropylalkohol; Tenside, insbesondere nichtionische Tenside, wie oben unter Emulgatoren 1. bis 18. beschrieben, Salze von Erdalkalimetallen, wie Calciumchlorid oder Magnesiumchlorid sowie Duftstoffe.

Die mit den erfindungsgemäßen Zusammensetzungen behandelten Textilien weisen einen verbesserten Weichgriff und eine verbesserte Hydrophilie auf als solche auf Basis von Esterquatverbindungen gemäß Stand der Technik.

Die erfindungsgemäßen β-Ketocarbonylquatverbindungen weisen eine überraschend hohe Hydrophilie auf im Vergleich zu den bekannten Esterquats, die hydrophob sind. Überraschend ist die ausgezeichnete Hydrophilie auch deswegen, weil Alkyl-Keten-Dimer-Dispersionen seit vielen Jahren zur kontrollierten Hydrophobierung von Papier weltweit eingesetzt werden (Papierleimung), und vergleichbare Anwendungen aus CH 388246 und US 5,028,236 bekannt sind.

In ihrer bevorzugten Amidform sind die erfindungsgemäßen β-Ketocarbonylquatverbindungen auch erheblich hydrolysestabiler als die handelsüblichen Esterquats, die daher nur in einem engen pH-Bereich gehandhabt und angewendet werden können.

Erfindungsgemäß können auch bakterizide β-Ketocarbonylquat-verbindungen mit einer längeren Alkylkette R hergestellt werden.

Die exzellent abgestufte Reaktivität des Alkyl-Keten-Dimers lässt es auch zu, durch den Einsatz von Aminoverbindung (b) mit OH-Gruppen, wie Dimethylaminoethanol, entsprechende Alkyl-Keten-Dimer-Esterquats herzustellen, falls Estergruppen zwischen dem Hydrophob- und dem Hydrophilteil gewünscht sind.

Die β-Ketoamidstruktur in den erfindungsgemäßen β-Ketocarbonyl-quatverbindungen hat den Vorteil, dass sie zur Komplexierung von Metallionen dienen kann. Hierdurch sind weitere Fixierungsmöglichkeiten auf Oberflächen gegeben.

### Herstellung der erfindungsgemäßen β-Ketocarbonylquat-verbindungen:

### Beispiel 1:

119 g Alkyl-Keten-Dimer mit einem Diketenequivalentgewicht von 570 g/Mol (Alkylrest R ca. 16 C-Atome, käuflich erwerblich als "AKD" bei Fa. Trigon Chemie GmbH) werden vorsichtig ohne Überhitzung aufgeschmolzen. Bei 63°C werden 21,4 g 3-Dimethylaminopropylamin langsam unter Kühlung und gutem Rühren zudosiert, so dass eine Innentemperatur von 75°C nicht überschritten wird. Wenige Minuten nach Beendigung der Zudosierung ist die Amidierung abgeschlossen. Es wird ein Amidoamin mit einer Aminzahl von 1,48 (theoretisch 1,49) erhalten, was eine praktisch 100 %-ige Ausbeute bedeutet.
Das erhaltene Amidoamin wird auf 100°C erwärmt und es werden insgesamt 37,2 g p-Toluolsulfonsäuremethylester (0,95 Mol je Mol tertiäre Aminogruppe in dem Amidoamin) über einen Zeitraum von 30 Minuten zugegeben. Man lässt weitere 2 Stunden bei 100°C nachreagieren und erhält 177,6 g eines Dialkylacetoacetamidoquats, das sich beim Abkühlen verfestigt. Das Produkt hat eine Aminzahl von 0,07, was einer Quaternierung von ca. 94 % entspricht. Dieser Wert stimmt gut mit der beabsichtigten 5 %-igen Unterdosierung des p-Toluolsulfonsäuremethylesters (MeOTs) überein.

### Beispiel 2:

Zu 119 g des in Beispiel 1 beschriebenen Alkyl-Keten-Dimer (570g/Mol Diketen) werden bei 61°C langsam insgesamt 39,2 g Bis-(3-dimethylamino-propyl)amin (käuflich erwerblich unter dem Handelsnamen "JEFFCAT Z 130" bei der Fa. Huntsman) dosiert. Die exotherme Reaktion der Amidierung ist kurz nach Beendigung der Dosierende abgeschlossen. Danach werden zu dem erhaltenen Amidoamin bei 100°C 74,5 g p-Toluolsulfonsäuremethylester (0,95 Mol je Mol tertiäre Aminogruppe in dem Amidoamin) portionsweise dosiert, wobei das Reaktionsgemisch 118°C erreicht. Bei 100°C lässt man 2 Stunden lang ausreagieren. Gemäß einer Aminzahl von 0,11 sind ca. 94 % der tertiären Aminogruppen quaterniert.

### Herstellung der Emulsion (A) aus den erfindungsgemäßen β-Ketocarbonylquatverbindungen:

5,0 g der in Beispiel 1 erhaltenen β-Ketocarbonylquatverbindung (AKD-Quat 1) werden in einem Becherglas vorsichtig aufgeschmolzen und mit 0,5 g Isopropanol versetzt. Danach werden bei 50°C 21,3 g Wasser zugegeben, sowie 1,0 g einer 80%-igen Lösung von Isotridecylalkohol-dekaethoxylat als Emulgator. Diese Mischung wird mit einem Magnetrührer intensiv gemischt. Es wird eine homogene, gelbliche Emulsion mit 18% Wirkstoffgehalt erhalten.

### Herstellung einer Vergleichsemulsion (V1) aus einer handelsüblichen Esterquatverbindung:

200 g einer handelsüblichen Esterquatlösung (90% Wirkstoff / 10% Isopropanol) erhältlich unter der Bezeichnung VK90 (Fa. Stepan) werden mit einem Flügelrührer in 800 ml Wasser eingerührt und auf einen pH-Wert von 3 eingestellt. Man erhält eine gelbliche Dispersion mit 18% Wirkstoffgehalt.

### Herstellung der Siliconöl-Emulsion (B):

In einem PC Laborsysteme Dissolver Typ LDV 1 der Fa. PC Laborsystem GmbH, Magden/Schweiz werden 26,40 g PEG-10 Isotridecylether und 14,00 g dest. Wasser vorgelegt und für zwei Minuten bei 500 U/min homogenisiert. Anschließend werden 480,00 g eines Siliconöls (Trimethylsilyl-endständiges Dimethylpolysiloxan) mit einer Viskosität von 350 mPa^{·}s (25°C) zugegeben und für 10 Minuten bei 2000 U/min homogenisiert. Danach werden in zwei Schritten 278 ml dest. Wasser zugegeben und bei 2000 U/min für insgesamt 15 Minuten homogenisiert. Anschließend werden 1,0 g einer 10 %-igen Lösung von Chlormethylisothiazolinon und Methylisothiazolinon als Konservierungsmittel zugesetzt.

### Herstellung der Mischung aus (A) und (B):

27 g Emulsion (A) aus der erfindungsgemäßen β-Ketocarbonylquat-verbindung wurde in ein Becherglas eingewogen, dann wurden 2,33 g der Siliconöl-Emulsion (B) dazugegeben. Mit dem Spatel wurde gerührt, bis alles homogen verteilt war. Anschließend wurde die Mischung in der Waschmaschine eingesetzt.

### Verwendung der Mischung als Weichspülers:

### Versuchsbeschreibungen:

Zur Ausrüstung in der Waschmaschine wird folgendes Gewebe verwendet: Frotteehandtuch (100 % Baumwolle, ca. 45 x 90 cm, ca. 200 g, ca. 500 g/m²)
Die zu testenden Gewebe werden vor der Verwendung zwei Mal bei 95°C mit Vollwaschmittel (z.B. Persil Waschpulver) in einer Haushaltswaschmaschine vom Typ Miele Novotronic W 941 gewaschen und anschließend auf einer Wäscheleine getrocknet.

### Weichgriffausrüstung im Spülgang:

Zur Ausrüstung wird ein Frotteehandtuch zusammen mit 2,0 kg Ballastwäsche (Baumwollgewebe) in die Waschmaschine gegeben. Direkt in die Trommel werden 80 g Vollwaschmittel (z.B. Persil Waschpulver) gegeben und die Waschmaschine im Vollwaschgang bei 40°C und einer Wasserhärte von 3°dH gestartet. Der Weichspüler wird von Hand zu Beginn des letzten Spülgangs direkt in die Waschtrommel gegeben. Die automatische Weichspülerdosierung über das Dosierfach wird nicht verwendet. Die Schleuderdrehzahl beträgt 1600 U/min. Anschließend werden die Gewebe auf einer Wäscheleine getrocknet. Danach erfolgt die Konditionierung über Nacht in einer Klimakammer bei 23°C und einer relativen Luftfeuchte von 60 %.

### Testmethode Weichgriff:

Den zu prüfenden Frotteehandtüchern werden Nummern von 1 bis n zugewiesen. In einem Blindversuch erhält eine Testperson das erste Handtuch als einen Standard (Nr. 1). Der Reihe nach vergleicht nun die Testperson den Weichgriff des Standardhandtuchs mit dem des Handtuches Nr. 2. Wird das Standardhandtuch als weicher empfunden erhält es die Note 1, ist es härter die Note 0 und wenn beide als gleich weich empfunden werden die Note 0,5. Dieses Ergebnis wird in eine Tabelle eingetragen. Anschließend wird Handtuch Nr. 1 mit Nr. 3 verglichen, dann mit Nr. 4 usw. bis n. Sodann wird das Handtuch Nr. 2 zum Standard und wird mit Nr. 1, 3, 4 bis n verglichen, usw. Die Einzelnoten werden zusammengezählt und ergeben die Weichgriffnote. Je höher die Note, umso weicher ist das Handtuch. Der Test wird mit mindestens 5 Testpersonen durchgeführt.

### Testmethode Tropfeneinsinkzeiten:

Das zu prüfende Gewebe wird über eine Petrischale mit einem Durchmesser von 140 mm gelegt, so dass die zu prüfende Stelle keinen Kontakt mit dem Untergrund hat. 70 µl vollentsalztes Wasser werden mit einer Pipette auf das Gewebe gegeben und die Stoppuhr gestartet. Gestoppt wird die Zeit, wenn der Wassertropfen vollständig eingezogen ist und darunter die ersten Gewebestrukturen erkennbar sind. Es werden immer Dreifachmessungen an verschiedenen Stellen des Gewebes durchgeführt. Als Tropfeneinsinkzeit wird der Mittelwert aus den drei Messwerten angegeben. Die maximale Testdauer beträgt 60 s, falls der Tropfen dann noch nicht vollständig eingezogen ist, wird als Tropfeneinsinkzeit > 60 s angegeben.

Im Spülgang wird eine Flottenkonzentration von 360 Gew.-ppm der organischen Quatverbindung (erfindungsgemäße β-Ketocarbonyl-quatverbindung bzw. Vergleichs-Esterquatverbindung) eingestellt, bzw. zusätzlich 100 Gew.-ppm des Siliconöls (Gew.-ppp jeweils bezogen auf den Wirkstoffgehalt).

Gemessen wird die Tropfeneinsinkzeit auf das Frotteehandtuch (Baumwollstoff). Der Weichgriff wird anhand der beschriebenen Testmethode ermittelt.
Die Ergebnisse sind in der Tabelle zusammenfasst.

**Tabelle:**

| Weichspülwirkstoff | Tropfeneinsinkzeit (Sekunden) | Weichgriffbeurteilung |
|---|---|---|
| Vergleich: | 26 | 1,0 |
| V1 | | |
| Vergleich: | 17 | 3,0 |
| V1 + Siliconöl B | | |
| erfindungsgemäß: | 1 | 6,0 |
| A + Siliconöl B | | |

Die Kombination der handelsüblichen Esterquat-Emulsion V1 mit der Siliconöl-Emulsion B ergibt bei der Hydrophilie bereits eine Verbesserung gegenüber dem Einsatz der reinen Esterquat-Emulsion V1. Die entscheidende wie auch überraschende Verbesserung wird aber erst durch den Austausch der handelsüblichen Esterquat-Emulsion V1 mit der erfindungsgemäßen β-Ketocarbonylquat-Emulsion A erreicht.

Beim Weichgriff ergibt der Zusatz der Siliconölemulsion zur handelsüblichen Esterquat-Emulsion V1 eine Verbesserung, aber erst der Austausch der handelsüblichen Esterquat-Emulsion V1 mit der erfindungsgemäßen β-Ketocarbonylquat-Emulsion A bringt die entscheidende Verbesserung.

## Patentansprüche

1. Zusammensetzungen enthaltend
(1) β-Ketocarbonylquatverbindungen, die eine oder mehrere β-Ketocarbonylgruppen der allgemeinen Formel
R-CH₂-(C=O)-CHR-(C=O)- (I)
und
eine oder mehrere quartäre Ammoniumgruppen enthalten, wobei
R gleich oder verschieden sein kann und einen aliphatischen Kohlenwasserstoffrest mit 6 bis 28 C-Atomen bedeutet, und
(2) bei 25°C und 1020 hPa flüssige Organopolysiloxane oder Siloxancopolymere.

2. Zusammensetzungen nach Anspruch 1, **dadurch gekennzeichnet, dass** es wässrige Dispersionen sind.

3. Zusammensetzungen nach Anspruch 1 oder 2, **dadurch gekennzeichnet, dass** sie enthalten
(A) Dispersionen von β-Ketocarbonylquatverbindungen enthaltend
(1) β-Ketocarbonylquatverbindungen,
(3) ggf. organische Lösungsmittel,
(4) ggf. Emulgator und
(5) Wasser
und
(B) Dispersionen von Organopolysiloxanen oder Siloxancopolymeren enthaltend
(2) bei 25°C und 1020 hPa flüssige Organopolysiloxane oder Siloxancopolymere,
(4') ggf. Emulgator und
(5') Wasser,
mit der Maßgabe, dass die Zusammensetzungen entweder als 2-Komponenten-Zusammensetzungen oder als Mischungen von (A) mit (B) in Form von 1-Komponenten-Zusammensetzung vorliegen.

4. Zusammensetzungen nach Anspruch 1, 2 oder 3, **dadurch gekennzeichnet, dass** die β-Ketocarbonylquatverbindungen (1) solche der allgemeinen Formel
[R³R⁴R⁵N⁽⁺⁾-R²-]ₐY-Z X⁽⁻⁾ (II),
sind, wobei
a 1 oder 2 ist, mit der Maßgabe, dass
wenn a=1, Y ein zweiwertiger Rest ist und
wenn a=2, Y ein dreiwertiger Rest ist,
Y einen zweiwertigen Rest der Formel -O-, -NH-, -NR¹-, oder einen dreiwertigen Rest der Formel =N- bedeutet,
X⁽⁻⁾ Gegenion zu der positiven Ladung am quartären Stickstoffatoms ist,
Z eine β-Ketocarbonylgruppe der Formel R-CH₂-(C=O)-CHR-(C=O)- (I) bedeutet,
R gleich oder verschieden sein kann und einen aliphatischen Kohlenwasserstoffrest mit 6 bis 28 C-Atomen, vorzugsweise 10 bis 26 C-Atomen, bevorzugt 12 bis 20 C-Atomen, bedeutet,
R¹ einen einwertigen Kohlenwasserstoffrest mit 1 bis 30 C-Atomen, vorzugsweise 1 bis 18 C-Atomen, bedeutet,
R² einen zweiwertigen C₁-C₁₈-Kohlenwasserstoffrest, der ein oder mehrere separate Sauerstoffatome enthalten kann, bedeutet,
R³ gleich oder verschieden ist und einen einwertigen Kohlenwasserstoffrest mit 1 bis 30 C-Atomen, vorzugsweise 1 bis 18 C-Atomen, bedeutet,
R⁴ gleich R³ ist oder einen Rest der Formel -R¹-Y-Z oder -R¹-N⁽⁺⁾R³₂R⁵ X⁽⁻⁾ bedeutet, oder R³ und R⁴ zusammen oder zwei Reste R³ zusammen einen zweiwertigen C₃-C₁₂-Kohlenwasserstoffrest, der gegebenenfalls ein O-Atom oder N-Atom enthalten kann, bedeuten und
R⁵ einen einwertigen Kohlenwasserstoffrest mit 1 bis 18 C-Atomen, vorzugsweise 1 bis 12 C-Atomen, bedeutet.

5. Zusammensetzungen nach Anspruch 4, **dadurch gekennzeichnet, dass** der Rest Y in Formel (II)
-NH-, -NR¹- oder =N- bedeutet,
wobei R¹ einen einwertigen Kohlenwasserstoffrest mit 1 bis 30 C-Atomen, vorzugsweise 1 bis 18 C-Atomen, bedeutet.

6. Zusammensetzungen nach einem der Ansprüche 1 bis 5, **dadurch gekennzeichnet, dass** die Organopolysiloxane (2) solche der allgemeinen Formel
R^{''}R'₂SiO(R'₂SiO)ₘSiR'₂R^{''} (VI)
sind, wobei
R' einen Kohlenwasserstoffrest mit 1 bis 18 C-Atomen, vorzugsweise einen C₁-C₁₈-Alkylrest, bevorzugt einen C₁-C₄-Alkylrest, bedeutet,
R^{''} einen Rest R', einen Hydroxyrest oder einen C₁-C₁₈-Alkoxyrest bedeutet und
m eine ganze Zahl von 20 bis 5000, vorzugsweise 100 bis 2000, bevorzugt 300 bis 2000, ist.
sind

7. Weichspülmittel enthaltend Zusammensetzungen nach einem der Ansprüche 1 bis 6.

## Claims

1. Compositions comprising
(1) β-ketocarbonylquats containing one or more β-ketocarbonyl groups of the general formula
R-CH₂-(C=O)-CHR-(C=O)- (I)
and
one or more quaternary ammonium groups, where
R is an aliphatic hydrocarbon radical of 6 to 28 carbon atoms and may be the same or different in each occurrence, and
(2) organopolysiloxanes or siloxane copolymers that are liquid at 25°C and 1020 hPa.

2. Compositions according to Claim 1, **characterized in that** they are aqueous dispersions.

3. Compositions according to Claim 1 or 2, **characterized in that** they comprise
(A) dispersions of β-ketocarbonylquats comprising
(1) β-ketocarbonylquats,
(3) optionally organic solvents,
(4) optionally an emulsifier, and
(5) water
and
(B) dispersions of organopolysiloxanes or siloxane copolymers comprising
(2) organopolysiloxanes or siloxane copolymers that are liquid at 25°C and 1020 hPa,
(4') optionally an emulsifier, and
(5') water,
with the proviso that the compositions are either 2-component compositions or mixtures of (A) with (B) in the form of a 1-component composition.

4. Compositions according to Claim 1, 2 or 3, **characterized in that** said β-ketocarbonylquats (1) have the general formula
[R³R⁴R⁵N⁽⁺⁾-R²-]ₐY-Z X⁽⁻⁾ (II),
wherein
a is 1 or 2, with the proviso that
when a=1, Y is a divalent radical and
when a=2, Y is a trivalent radical,
Y is a divalent radical of the formula -O-, -NH-, -NR¹-, or a trivalent radical of the formula =N-,
X⁽⁻⁾ is a counter-ion to the positive charge on the quaternary nitrogen atom,
Z is a β-ketocarbonyl group of the formula R-CH₂-(C=O)-CHR-(C=O)- (I),
R is an aliphatic hydrocarbon radical of 6 to 28 carbon atoms, preferably 10 to 26 carbon atoms, more preferably 12 to 20 carbon atoms, and may be the same or different in each occurrence,
R¹ is a monovalent hydrocarbon radical of 1 to 30 carbon atoms, preferably 1 to 18 carbon atoms,
R² is a divalent C₁-C₁₈ hydrocarbon radical which may contain one or more separate oxygen atoms,
R³ is a monovalent hydrocarbon radical of 1 to 30 carbon atoms, preferably 1 to 18 carbon atoms, and is the same or different in each occurrence,
R⁴ is R³ or a radical of the formula -R¹-Y-Z or -R¹-N⁽⁺⁾R³₂R⁵ X⁽⁻⁾,
or R³ and R⁴ together or two R³ radicals together are a divalent C₃-C₁₂ hydrocarbon radical which may optionally contain an oxygen atom or a nitrogen atom, and
R⁵ is a monovalent hydrocarbon radical of 1 to 18 carbon atoms, preferably 1 to 12 carbon atoms.

5. Compositions according to Claim 4, **characterized in that** Y in formula (II) is
-NH-, -NR¹- or =N-,
where R¹ is a monovalent hydrocarbon radical of 1 to 30 carbon atoms, preferably 1 to 18 carbon atoms.

6. Compositions according to any of Claims 1 to 5, **characterized in that** said organopolysiloxanes (2) have the general formula
R^{''}R'₂SiO(R'₂SiO)ₘSiR'₂R^{''} (VI),
where
R' is a hydrocarbon radical having 1 to 18 carbon atoms, preferably a C₁-C₁₈-alkyl radical, more preferably a C₁-C₄-alkyl radical,
R^{''} is R', a hydroxyl radical or a C₁-C₁₈-alkoxy radical and
m is an integer from 20 to 5000, preferably 100 to 2000, more preferably 300 to 2000.

7. Fabric conditioner comprising compositions according to any of Claims 1 to 6.

## Revendications

1. Compositions contenant
(1) des composés de ß-cétocarbonylquat, qui contiennent un ou plusieurs groupes ß-cétocarbonyle de formule générale
R-CH₂- (C=O) -CHR- (C=O)- (I)
et
un ou plusieurs groupes d'ammonium quaternaire,
R pouvant être identique ou différent et signifiant un radical hydrocarboné aliphatique comprenant 6 à 28 atomes de carbone, et
(2) des organopolysiloxanes ou des copolymères de siloxane liquides à 25°C et à 1020 hPa.

2. Compositions selon la revendication 1, **caractérisées en ce qu'**il s'agit de dispersions aqueuses.

3. Compositions selon la revendication 1 ou 2, **caractérisées en ce qu'**elles contiennent
(A) des dispersions de composés ß-cétocarbonylquat contenant
(1) des composés ß-cétocarbonylquat,
(3) le cas échéant des solvants organiques,
(4) le cas échéant des émulsifiants et
(5) de l'eau et
(B) des dispersions d'organopolysiloxanes ou de copolymères de siloxane contenant
(2) des organopolysiloxanes ou des copolymères de siloxane liquides à 25°C et à 1020 hPa,
(4') le cas échéant des émulsifiants et
(5') de l'eau,
à condition que les compositions se trouvent soit sous forme de composition à 2 composants, soit, comme mélanges de (A) et de (B), sous forme d'une composition à 1 composant.

4. Compositions selon la revendication 1, 2 ou 3, **caractérisées en ce que** les composés ß-cétocarbonylquat (1) sont ceux de formule générale
[R³R⁴R⁵N⁽⁺⁾-R²-]ₐY-Z X⁽⁻⁾ (II),
où
a vaut 1 ou 2, à condition que
lorsque a=1, Y représente un radical divalent et
lorsque a=2, Y représente un radical trivalent,
Y représente un radical divalent de formule -O-, -NH-, -NR¹-, ou un radical trivalent de formule =N-,
X⁽⁻⁾ représente un contre-ion par rapport à la charge positive sur l'atome d'azote quaternaire,
Z représente un groupe ß-cétocarbonyle de formule R-CH₂-(C=O)-CHR-(C=O)- (I),
R peut être identique ou différent et signifie un radical hydrocarboné aliphatique comprenant 6 à 28 atomes de carbone, de préférence de 10 à 26 atomes de carbone, de préférence 12 à 20 atomes de carbone,
R¹ signifie un radical hydrocarboné monovalent comprenant 1 à 30 atomes de carbone, de préférence 1 à 18 atomes de carbone,
R² signifie un radical hydrocarboné en C₁-C₁₈ divalent, qui peut contenir un ou plusieurs atomes d'oxygène séparés,
R³ est identique ou différent et signifie un radical hydrocarboné monovalent comprenant 1 à 30 atomes de carbone, de préférence 1 à 18 atomes de carbone,
R⁴ est identique à R³ ou signifie un radical de formule -R¹-Y-Z ou -R¹-N⁽⁺⁾R³₂R⁵ X⁽⁻⁾, ou R³ et R⁴ ensemble ou deux radicaux R³ ensemble signifient un radical hydrocarboné en C₃-C₁₂ divalent, qui peut le cas échéant contenir un atome d'oxygène ou un atome d'azote et
R⁵ signifie un radical hydrocarboné monovalent comprenant 1 à 18 atomes de carbone, de préférence 1 à 12 atomes de carbone.

5. Compositions selon la revendication 4, **caractérisées en ce que** le radical Y dans la formule (II) signifie
-NH-, -NR¹- ou =N-,
R¹ signifiant un radical hydrocarboné monovalent comprenant 1 à 30 atomes de carbone, de préférence 1 à 18 atomes de carbone.

6. Compositions selon l'une quelconque des revendications 1 à 5, **caractérisées en ce que** les organopolysiloxanes (2) sont ceux de formule générale
R"R'₂SiO(R'₂SiO)ₘSiR'₂R" (VI)
où
R' signifie un radical hydrocarboné comprenant 1 à 18 atomes de carbone, de préférence un radical C₁-C₁₈-alkyle, de préférence un radical C₁-C₄-alkyle,
R'' signifie un radical R', un radical hydroxy ou un radical C₁-C₁₈-alcoxy et
m représente un nombre entier de 20 à 5000, de préférence de 100 à 2000, de préférence de 300 à 2000.

7. Agent assouplissant contenant des compositions selon l'une quelconque des revendications 1 à 6.
